# EUROPÄISCHE PATENTSCHRIFT

(11) **EP 0 418 355 B1**
(45) Veröffentlichungstag und Bekanntmachung des Hinweises auf die Patenterteilung: **17.08.1994**
(21) Anmeldenummer: 90905161.7
(22) Anmeldetag: 04.04.1990
(51) Int. Cl.: C12N 11/14, C07K 17/14, C12M 1/40

(54) **VERFAHREN ZUM IMMOBILISIEREN VON PROTEINEN, PEPTIDEN, COENZYMEN OD.DGL. AN EINEM TRÄGER**
PROCESS FOR IMMOBILIZING PROTEINS, PEPTIDES, COENZYMES, ETC. ON A SUBSTRATE
PROCEDE POUR IMMOBILISER DES PROTEINES, DES PEPTIDES, DES COENZYMES ETC. SUR UN SUPPORT

(30) Priorität: 04.04.1989 AT 786/89; 10.05.1989 AT 1119/89
(43) Veröffentlichungstag der Anmeldung: 27.03.1991
(73) Patentinhaber: PITTNER, Fritz, Ass.Prof.Doz.Dr., 1235 Wien (AT); SCHALKHAMMER, Thomas, Mag.Dr., A-3100 St.Pölten (AT); Urban, Gerald Dr., A-1020 Wien (AT); MANN-BUXBAUM, Eva, Mag., A-1140 Wien (AT)
(72) Erfinder: PITTNER, Fritz, Ass.Prof.Doz.Dr., 1235 Wien (AT); SCHALKHAMMER, Thomas, Mag.Dr., A-3100 St.Pölten (AT); Urban, Gerald Dr., A-1020 Wien (AT); MANN-BUXBAUM, Eva, Mag., A-1140 Wien (AT)
(74) Vertreter: Kunz, Ekkehard, Dr.
(86) Internationale Anmeldenummer: AT9000026
(87) Internationale Veröffentlichungsnummer: WO9012092

(56) Entgegenhaltungen:
- GB-A- 2 163 434
- Derwent's abstract No. 564 25 D/31, SU 777 041, publ. week 8131 DAIYA YA
- Methods in Enzymology, vol. XLIV, Klaus Mosbach; "Immobilized Enzymes", Academic Press, New York, San Francisco, LOndon, 1976, p.134-149.

## Beschreibung

Die Erfindung bezieht sich auf ein Verfahren zum Immobilisieren von Proteinen, Peptiden, Liganden, Coenzymen oder Redoxmediatoren an einem Träger, welcher Amino-, Mercapto- oder Hydroxygruppen aufweist. Derartige Immobilisate sind unter anderem als Enzymimmobilisate bekannt, welche in Bioreaktoren oder im Bereich der Biosensorik eingesetzt werden können. Enzymimmobilisate der eingangs genannten Art enthalten beispielsweise β-Glucosidasen, Lipasen, Esterasen, Phosphatasen, Oxidreduktasen oder Naringinasen und werden für die Behandlung von Lebensmitteln, die Reinigung von Abwässern, biokatalysierte organische Synthesen, Bioanalytik und Medizintechnik herangezogen. Bei den derzeit bekannten und verwendeten Immobilisierungsmethoden können Anzahl und räumliche Verteilung der zur Kopplung geeigneten reaktiven Gruppen nur in seltenen Fällen und nur unzureichend gesteuert werden. Auch ist es meist nicht möglich, Trägermaterialien mit Amino-, Mercapto- bzw. Hydroxygruppen mit einem einzigen Verfahren zu aktivieren. Auch die Lagerung des aktivierten Trägermaterials ist bei den meisten Verfahren nicht möglich.

Immobilisate der eingangs genannten Art werden zumeist unter Verwendung von Glutardialdehyd hergestellt, wobei zunächst je nach Art des Trägers der Träger soweit derivatisiert werden muß, daß er primäre Aminogruppen aufweist, welche in der Folge mit Glutardialdehyd und in weiterer Folge mit den zu immobilisierenden Proteinen, Peptiden oder Coenzymen umgesetzt werden. Derivatisierte Träger, welche mit Glutardialdehyd umgesetzt wurden, lassen sich ohne Aktivitätsverlust nicht trocknen.

Die Erfindung zielt nun darauf ab, ein Verfahren der eingangs genannten Art zu schaffen, bei welchem ein derivatisierter Träger, mit Amino- aber auch Mercapto- und Hydroxygruppen nach einer Kopplung unmittelbar mit den zu immobilisierenden Proteinen, Peptiden, Liganden oder Coenzymen umgesetzt werden kann, ohne Aktivitätsverlust getrocknet werden kann, und mit welchem auch dünne Schichten an Protein, Peptiden oder Coenzymen, insbesondere Monolayers, bei gleichzeitig hoher mechanischer Beanspruchbarkeit immobilisiert werden können. Zur Lösung dieser Aufgabe besteht das erfindungsgemäße Verfahren im wesentlichen darin, daß der Träger mit einer Verbindung der allgemeinen Formel (I) oder (IA) 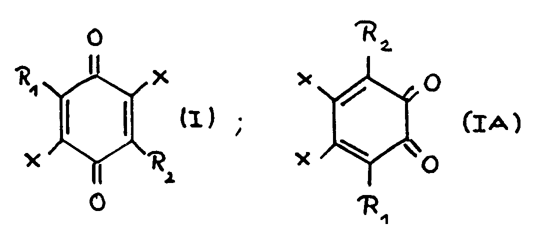
in welchen X Halogen bedeutet und R₁, R₂ gleich oder ungleich sind und X, R, COR, COOR, worin R C₁ - C₈ Alkyl bedeutet, COOH, CNS, N₃ oder CN bedeuten, umgesetzt und gewünschtenfalls getrocknet wird, worauf die zu immobilisierenden Peptide, Proteinen oder Coenzyme mit dem zur C=O Gruppe ortho- oder paraständigen, freien Halogen der Verbindung der allgemeinen Formel (I) oder (IA) umgesetzt werden. Die Verbindung der allgemeinen Formel (I) oder (IA) bildet hiebei zunächst über einen Substituenten X eine kovalente Bindung mit den Amino-, Mercapto- oder Hydroxygruppen des Träger aus, wodurch eine besonders stabile Kupplung erzielt wird. Nun kann der zweite, durch die erste Kopplung aktivierte, Substituent X mit Amino-, Hydroxy- oder Mercaptogruppen des zu immobilisierenden Reaktanten zur Reaktion gebracht werden, wobei eine besonders stabile kovalente Kupplung erzielt wird. Die Kupplungsreaktion zwischen der Verbindung der allgemeinen Formel (I) oder (IA) und dem Träger bzw. der zu immobilisierenden Verbindung ist sehr in hohem Maße von der Reaktivität der jeweiligen Substituenten abhängig. So ist bei Verbindungen der allgemeinen Formel (I) oder (IA) in welcher X = R₁ = R₂ die Reaktivität entsprechend der folgenden Reihe abnehmend
X = F, Cl, Br > J, N₃, COOH, COR, COOR, NO₂, > CN, CNS, R, wobei R C₁ - C₈ Alkyl bedeutet. Die bevorzugten Substituenten der Verbindungen der Formel (I) oder (IA) sind daher Fluor, Chlor und Brom. Als Modellreaktion kann daher die Kopplung von o-Chloranil bzw. o-Bromanil mit einem Aminoträgermaterial wie beispielsweise aminosililierte Metall- oder Glasoberflächen angesehen werden. Auch die Reaktivität des Trägers ist von den jeweiligen Substituenten abhängig und nimmt entsprechend der folgenden Reihe ab

NH₂ > NH, SH » OH.

Die Reaktivität des so aktivierten Trägermaterials ist für die Kopplung von Amino- und Mercaptogruppen deutlich größer als jene für die Kopplung von Hydroxygruppen. Am bevorzugtesten ist daher die Umsetzung eines Trägers welcher eine primäre Aminogruppe trägt mit einer Verbindung der Formel I, in welcher die Substituenten X, R₁ und R₂ gleich sind und jeweils Fluor, Chlor oder Brom bedeuten.

Das freie, zu einer Carbonylgruppe ortho- oder paraständige Halogen kann in der Folge wiederum durch Ausbildung kovalenter Bindungen mit Aminogruppen, Hydroxygruppen bzw. gegebenenfalls Mercaptogruppen der zu immobilisierenden Proteine, Peptide oder Coenzyme unter Ausbildung kovalenter Bindungen umgesetzt werden, wodurch ohne weiteres auch Monolayers mit hoher mechanischer Belastbarkeit hergestellt werden können. Die Anzahl und räumliche Anordnung der reaktiven Gruppen kann hiebei durch die photochemische Reaktion so gesteuert werden, daß auch die gezielte Anordnung von unterschiedlichen Monolayerstrukturen auf einem Träger bzw. die gezielte Immobilisierung von Multilayerschichten möglich ist. Die Umsetzung des Kopplungsagens mit den Proteinen Peptiden und Coenzymen od.dgl. ist ebenfalls von den jeweiligen reaktiven Gruppen der Proteine, Peptide oder Coenzyme abhängig. Bei der Umsetzung dieser Verbindungen in Wasser oder Pufferlösungen hat sich folgende Reaktivitätsreihe

NH₂ > NH ∼ SH » OH

ergeben.

Die Verwendung einer Verbindung der allgemeinen Formel (I) oder (IA) für die Immobilisierung von Proteinen, Peptiden, Coenzymen oder Liganden an einem Träger bietet darüber hinaus den Vorteil, daß die Verbindung der allgemeinen Formel (I) oder (IA) in besonders einfacher Weise über bestimmte Bereiche der Oberfläche des Trägers desaktiviert werden kann. Die Desaktivierung kann in einfacher Weise durch photochemische Umsetzung oder durch Umsetzung mit Basen vorgenommen werden, wodurch insbesondere bei selektiver photochemischer Desaktivierung überaus kleine Bauteile mit der gewünschten Kopplungsaktivität hergestellt werden können. Derartige kleine Bauteile eignen sich in der Folge beispielsweise auch für den Einsatz als Biosensoren, aber auch für chemische und biochemische Detektionssysteme auf Teststreifenbasis.

Als Träger kommen hiebei übliche Träger für die Herstellung von Immobilisaten in Betracht, wobei insbesondere für die Verwendung als elektrochemische Biosensoren leitfähige Schichten oder leitfähige Träger aus Metallen, wie beispielsweise Gold, Platin, Palladium, Rhodium oder Kohlenstoff, in Betracht kommen.

In besonders vorteilhafter Weise kann im Rahmen des erfindungsgemäßen Verfahrens für die Immobilisierung als Verbindung der Formel (I) 2,3,5,6-Tetrachlorcyclohexadien-1,4-dion eingesetzt werden und für die Immobilisierung als Verbindung der Formel (IA) 3,4,5,6-Tetrachlorcyclohexadien-1,2-dion oder 3,4,5,6-Tetrabromcyclohexadien-1,2-dion eingesetzt werden. Die zusätzlichen Substituenten, wie weitere Halogenatome, COOH, COR, COOR, CNS, NO₂, N₃ oder CN, welche als desaktivierende Substituenten bei der elektrophilen Substitution von Aromaten bekannt sind, aktivieren die an der Kupplungsreaktion zwischen Träger und den Proteinen, Peptiden, Coenzymen oder Liganden teilnehmenden paraständigen Halogenatome weiter, so daß eine besonders stabile, kovalente Bindung erzielt werden kann. Diese Transaktivierung für die Kupplung des Trägers mit den zu immobilisierenden Substanzen kann durch Wahl der weiteren Substituenten, insbesondere durch die Auswahl von Halogen, wie Cl, Br oder Gruppen wie z.B. 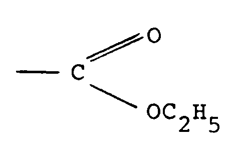
entsprechend gesteuert werden, wobei auch Aktivitätsverbesserungen durch entsprechende sterische Ausrichtung der zu immobilisierenden Substanzen erzielt werden können.

In besonders einfacher Weise kann die Umsetzung des Trägers mit Verbindungen der allgemeinen Formel (I) oder (IA) in organischen oder organisch wäßrigen Mischphasen vorgenommen werden, wobei die Umsetzung des Trägers mit Verbindungen der allgemeinen Formel (I) oder (IA) in wasserfreien, organischen Lösungsmitteln, insbesondere Toluol, besonders bevorzugt ist. Insbesondere die Umsetzung der Verbindung der allgemeinen Formel (I) oder (IA) mit dem Träger kann hiebei zur Beschleunigung der Reaktion ohne weiteres bei erhöhten Temperaturen vorgenommen werden, wobei bei Temperaturen zwischen -10°C und Rückflußtemperatur, vorzugsweise 20-70°C, gearbeitet werden kann. Für die Immobilisierung der Proteine, Peptide, Coenzyme oder Liganden muß naturgemäß die Temperatur in einer Weise beschränkt werden, daß die zu immobilisierenden Substanzen nicht thermisch denaturiert werden können.

Als Proteine können mit Vorteil im Rahmen des erfindungsgemäßen Verfahrens Redoxenzyme, wie z.B. Glucoseoxidase, Galactoseoxidase, Aminosäureoridase, Xanthinoxidase, Cholesterinoxidase, Uricase und Ascorbatoxidase bzw. deren Apoenzyme eingesetzt werden. Derartige Redoxenzyme eignen sich besonders für die Herstellung von Biosensoren, wobei die entsprechende enzymkatalysierte Redoxreaktion, welche die Redoxenzyme bewirken, über Folgeprodukte z.B. H₂O₂ elektrochemisch gemessen werden kann. Es sind auf diese Weise miniaturisierte Biosensoren für die Blutzuckerbestimmung unter Verwendung von Glucoseoxidase herstellbar. Mit Biosensoren auf der Basis von Xanthinoxidase kann in besonders einfacher Weise die Frische von Lebensmitteln, wie beispielsweise Fischen, bestimmt werden. Analog lassen sich eine Reihe von Biosensoren mit derartigen Redoxenzymen herstellen, wobei für die Herstellung derartiger Biosensoren als besonderer Vorteil der Verwendung der Verbindung der allgemeinen Formel (I) oder (IA) der Umstand hinzukommt, daß sich Teilbereiche der Oberfläche des Trägers nach der Umsetzung mit der Verbindung der allgemeinen Formel (I) oder (IA) photochemisch desaktivieren lassen. Auf diese Weise lassen sich exakt definierte, reaktive Oberflächen schaffen, und es lassen sich insbesondere auch mehrschichtige, kleinbauende Biosensoren verwirklichen, bei welchen Teilen der Oberfläche unterschiedlicher Funktionen unterschiedlich selektive Empfindlichkeit zukommt. Mit Vorteil kann hiefür so vorgegangen werden, daß die Oberfläche des Umsetzungsproduktes mit der Verbindung der allgemeinen Formel (I) oder (IA) teilweise durch photochemische Reaktion oder durch Anwendung alkalischer Reagentien bzw. niedermolekularer Amine oder Mercaptane desaktiviert wird, worauf die Umsetzung mit den Peptiden, Proteinen, Coenzyme oder Liganden mit den verbleibenden Teilen der Oberfläche vorgenommen wird, wobei zur Erzielung eines mehrschichtigen oder multifunktionialen Biosensors in einfacher Weise so vorgegangen werden kann, daß das Immobilisat neuerlich mit einer Verbindung der allgemeinen Formel (I) oder (IA) umgesetzt wird, worauf gegebenenfalls nach teilweiser Desaktivierung der Oberfläche neuerlich Peptide, Proteine oder Coenzyme immobilisiert werden. Für die Herstellung eines Biosensors erfolgt die Immobilisierung mit Vorteil auf einem mit Elektroden versehenen Träger, und zur weiteren Erhöhung der mechanischen Belastbarkeit kann mit Vorteil so vorgegangen werden, daß das Immobilisat für die Verwendung als Biosensor mit einer physiologisch verträglichen Deckschicht, insbesondere mit einer negativen Raumladung, versehen wird.

Insbesondere die Ausbildung einer Außenseite der Deckschicht mit einer negativen Raumladung hat hiebei den Vorteil, daß die Bestimmung beeinträchtigende Nebenreaktionen hintangehalten werden können. So wird insbesondere die Glucosebestimmung mit Glucoseoxidase durch Ascorbat und Citrat gestört, wobei eine negative Ladung der Außenseite einer Deckschicht einem Eindringen derartiger Anionen entgegenwirkt und auf diese Weise eine Verzerrung der Meßwerte vermieden wird.

Die Verwendung der Verbindung der allgemeinen Formel (I) oder (IA) für die Herstellung von Biosensoren im obigen Sinne hat hiebei gegenüber anderen Methoden der Immobilisierung, insbesondere gegenüber einer Immobilisierung mit Glutardialdehyd, den wesentlichen Vorteil, daß die Kupplungsschichte Redoxaktivität zeigt, so daS die Verbindung der allgemeinen Formel (I) oder (IA) als redoxübertragende Zwischenschicht zur Übertragung von Elektronen Verwendung finden kann. Auf diese Weise wird die Empfindlichkeit eines unter Verwendung einer Verbindung der allgemeinen Formel (I) oder (IA) hergestellten Biosensors wesentlich gesteigert. Die Verbindungen der Formal (IA), nämlich o-Chinone, sind durch ihre Redoxaktivität für den Elektronentransfer zwischen Redoxenzymen und der Elektrodenoberfläche einsetzbar. Weiters kann die o-Chinonstruktur mit aromatischen o-Diaminen zu Phenanzinstrukturen gekoppelt werden, die sich ebenfalls als Redoxmediatoren für den Elektronentransfer zwischen Elektrode und Cofaktoren eignen.

Neben der Verwendung der erfindungsgemäß hergestellten Immobilisate für die Herstellung von Biosensoren lassen sich auch Immobilisate für diagnostische Zwecke, wie beispielsweise Teststreifen, Immunsensonren dadurch herstellen, daß Antigene oder Antikörper zum Immobilisieren eingesetzt werden. Schließlich ist es mit Rücksicht auf die besonders hohe mechanische Stabilität des Immobilisates, möglich auch biotechnologisch in Bioreaktoren, Fermentatoren, bioorganischen Synthesen einzusetzende Immobilisate herzustellen, wobei vorzugsweise α oder β-Glucosidasen, Glycosidasen, Galaktosidasen, Lipasen, Pectinasen, Esterasen, Penicillininase, Ammoniaklyasen, Urease und/oder Phosphatasen eingesetzt werden. Derartige Immobilisate eignen sich für die Bioprozeßtechnik, Lebensmitteltechnik, Abwasser- und Umwelttechnologie.

Zur Erzielung von Multilayers hat es sich als besonders vorteilhaft erwiesen, das Verfahren so zu führen, daß Proteine, Peptide, Coenzyme od.dgl. mit einer wenigstens äquimolaren Menge einer Verbindung der Formel I oder (IA) 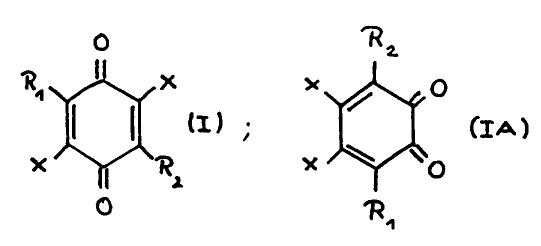
in welchen X Halogen bedeutet und R₁, R₂ gleich oder ungleich sind und X, R, COR, COOR, worin R C₁ - C₈ Alkyl bedeutet, COOH, CNS, N₃ oder CN bedeuten, umgesetzt werden und gegebenenfalls getrocknet werden, worauf das erhaltene Umsetzungsprodukt über ein freies zur C=O Gruppe ortho- oder paraständiges Halogen der Verbindung der Formel I mit dem Träger gekuppelt wird. Durch die Umsetzung einer Verbindung der Formel (I) oder (IA) mit Proteinen, Peptiden, Coenzymen oder Liganden ist es möglich über Brücken der Verbindung der Formel (I) oder (IA) quervernetzte Proteine oder Peptide zu erhalten, welche über ein freies nicht vernetztes paraständiges Halogen an den Träger gebunden werden können. Auf diese Weise ist es möglich, unmittelbar innerhalb eines Reaktionsschrittes Multilayers von auf einem Träger immobilisierten Enzymen zu erhalten.

Es ist im Rahmen des erfindungsgemäßen Verfahrens auch möglich, die Reihenfolge der Aktivierungsschritte umzukehren und zuerst die Proteine, Peptide, Liganden, Effektoren, welche Amino- Mercapto- bzw. Hydroxygruppen aufweisen, mit einer Verbindung der allgemeinen Formel (I) oder (IA) zur Reaktion zu bringen und dann die so aktivierten Moleküle an einen Träger zu binden. Durch die bifunktionellen Eigenschaften der Verbindung der allgemeinen Formel (I) oder (IA) werden Proteine, Peptide oder Liganden zu polymeren Makromolekülen vernetzt, welche in der Form ebenfalls als Immobilisate einsetzbar sind, die sodann mit den freien reaktiven Gruppen zusätzlich an ein Trägermaterial gebunden werden können. Für Spezialprobleme kann als Trägermaterial auch ein weiteres Makromolektül, wie beispielsweise ein Dextran, Xanthan, Polyethylenglycol, Polyethylenimin oder Trägerprotein dienen. Durch Wahl der Reaktionsbedingungen kann der Polymerisationsgrad und der Immobilisierungstyp gesteuert werden.

Die Erfindung wird nachfolgend an Hand von Ausführungsbeispielen näher erläutert.

### Beispiel 1: Derivatisierung von mikroporösem Glas

### A. wäßrige Silanisierung:

100 g mikroporöses Glas werden mit einer 10 %-igen wäßrigen 3-(Triethoxysilyl)-propylamin Lösung versetzt. Innerhalb der ersten 30 Minuten muß der pH der Reaktionslösung ständig kontrolliert und auf pH 3,5 eingestellt werden. Dieser Ansatz wird 4 Stunden bei Raumtemperatur gerührt. Anschließend wird das mikroporöse Glas von der Reaktionslösung getrennt und 5-mal mit je 100 ml Wasser gewaschen und bei 110°C getrocknet.

### B. organische Silanisierung:

100 g mikroporöses Glas werden mit einer 10 %-igen 3-(Triethoxysilyl)-propylamin Lösung in Toluol versetzt. Dieser Ansatz wird 5 Stunden bei Raumtemperatur gerührt. Anschließend wird das silanisierte mikroporöse Glas von der Reaktionslösung getrennt und 5-mal mit je 50 ml Toluol gewaschen.

### C. Aktivierung:

a) Das nach dem Verfahren A oder B hergestellte mikroporöse aminierte Glas wird mit einer 2,5 %-igen 2,3,5,6-Tetrachlorcyclohexadien-1,4-dion-Toluol Lösung versetzt und 30 Minuten bei 40°C gerührt. Nach Beendigung der Reaktion wird das Glas von der Redktionslösung getrennt und 5-mal mit je 50 ml Toluol und 2-mal mit je 100 ml Aceton gewaschen. Anschließend wird das derivatisierte mikroporöse Glas mit Wasserstrahlvakuum trocken gesaugt und in diesem Zustand gelagert.
   Die Derivatisierung der silanisierten Oberflächen wurde auch unter Verwendung von 2,3,5,6-Tetrabrom- bzw. 2,3,5,6-Tetrafluor- bzw. 2,3,5,6-Tetraiodocyclohexadien-1,4-dion durchgeführt.
b) Das nach dem Verfahren A oder B hergestellte mikroporöse aminierte Glas wird mit einer 2 %igen 3,4,5,6-Tetrachlorcyclohexadien-2,1-dion Lösung in Toluol versetzt und 30 Minuten bei 25°C durchmischt. Nach Beendigung der Reaktion wird das Glas von der Reaktionslösung getrennt und 5 mal mit je 50 ml Toluol und 2 mal mit je 100 ml Aceton gewaschen. Helle Lichteinstrahlung und erhöhte Temperaturen sind während der Aktivierung und auch danach solange noch nicht gekoppelt wurde zu vermeiden. Anschließend wird das derivatisierte mikroporöse Glas im Wasserstrahlvakuum getrocknet und in diesem Zustand unter Lichtausschluß bei 4°C gelagert.
   Die Derivatisierung der silanisierten Oberflächen wurde auch unter Verwendung von
   3,4,5,6-Tetrabromcyclohexadien-1,2-dion bzw.
   3,4,5,6-Tetrafluorcyclohexadien-1,2-dion durchgeführt.

### Beispiel 2: Derivatisierung von Platin, Palladium- bzw. Rhodiumoberflächen

### A. chemische Oxidation der Oberflächen:

Die Pt, Pd- bzw. Rh-Oberflächen werden 20 Stunden in eine 15 %-ige wäßrige HNO₃ Lösung bei Raumtemperatur gelegt und anschließend mehrmals mit Wasser gewaschen.

### B. elektrochemische Oxidation der Oberflächen:

Die Pt, Pd- bzw.Rh-Oberflächen werden in einer wäßrigen 10 %-igen HNO₃, 2,5 %-igen Cr₂O₇-Lösung 15 Sekunden unter kräftiger Sauerstoffentwicklung oxidiert.

### C. Silanisierung und Aktivierung:

Die nach dem Verfahren A oder B oxidierten Pt, Pd- bzw. Rh-Oberflächen werden mehrmals mit Aceton gespült, bei Raumtemperatur getrocknet und anschließend in eine 10 %-ige 3-(Triethoxysilyl)-propylamin-Toluol Lösung 30 Minuten bei 60°C gelegt.
a) Die silanisierten Oberflächen werden danach mit Toluol gewaschen und 30 Minuten in eine 2 %-ige 2,3,5,6-Tetrachlorocyclohexadien-1,4-dion-Toluol Lösung bei 40°C gelegt. Nach Beendigung der Reaktion wurden die Oberflächen mehrmals mit Toluol und zuletzt mit Aceton gespült. Die derivatisierten Pt bzw. Pd-Oberflächen werden in trockenem Zustand unter Lichtabschluß gelagert.
b) Die silanisierten Oberflächen werden mit Toluol gewaschen und 20 Minuten bei 110°C getrocknet. Sodann werden die aminierten Metalloberflächen in eine 2 %ige Lösung von 3,4,5,6-Tetrachlorcyclohexadien-1,2-dion in Toluol gelegt und bei 25°C 20 Minuten darin belassen. Nach Beendigung der Reaktion werden die Elektroden mit Aceton gespült und unter Lichtabschluß bei 4°C gelagert. Während des gesamten Aktivierungsprozesses muß bei stark gedämpftem Licht, am besten Rotlicht, gearbeitet werden.

### Beispiel 3: Derivatisierung von porösem Silikatmaterial

100 g poröses Silikatmaterial werden mit einer 10 %-igen 3-(Triethoxysilyl)-propylamin-Toluol Lösung versetzt. Dieser Ansatz wird 1 Stunde bei 60°C gerührt. Anschließend wird das silanisierte poröse Silikatmaterial von der Reaktionslösung getrennt und 5-mal mit je 50 ml Toluol gewaschen.
a) Danach wird das poröse Silikatmaterial mit einer 1,5%-igen 2,3,5,6-Tetrachlorcyclohexadien-1,4-dion-Toluol Lösung versetzt und 30 Minuten bei 40°C gerührt. Nach Beendigung der Reaktionsdauer wird das Silikatmaterial von der Reaktionslösung getrennt und 5-mal mit je 50 ml Toluol und 2-mal mit je 100 ml Aceton gewaschen. Anschließend wird das derivatisierte Silikat mit Wasserstrahlvakuum trocken gesaugt und in diesem Zustand gelagert.
   Die Derivatisierung von porösem Silikatmaterial wurde auch unter Verwendung von 2,3,5,6-Tetrabromcyclohexadien-1,4-dion, 2,3,5,6-Tetraiodcyclohexadien-1,4-dion durchgeführt.
b) Danach wird das poröse Silikatmaterial mit einer 1 %igen 3,4,5,6-Tetrachlorcyclohexadien-1,2-dion Lösung in Toluol versetzt und 30 Minuten bei 25°C gerührt. Nach Beendigung der Reaktion wird das Silikatmaterial von der Reaktionslösung getrennt und 5 mal mit je 50 ml Acton gewaschen. Anschließend wird das derivatisierte Silikat im Wasserstrahlvakuum getrocknet und in diesem Zustand unter Lichtabschluß bei 4°C gelagert.
   Die Derivatisierung der silanisierten Oberflächen wurde auch unter Verwendung von
   3,4,5,6-Tetrabromcyclohexadien-1,2-dion bzw.
   3,4,5,6-Tetrafluorcyclohexadien-1,2-dion durchgeführt.

### Beispiel 4: Photodesaktivierung von 2,3,5,6-Tetrachlor- bzw. 2,3,5,6-Tetrabrom- bzw. 2,3,5,6-Tetrafluor- bzw. 2,3,5,6-Tetraiodcyclohexadien-1,4-dion sowie 3,4,5,6-Tetrachlor- bzw. 3,4,5,6-Tetrafluor- bzw. 3,4,5,6-Tetrabromcyclohexadien-1,2-dion derivatisierten Oberflächen

Die derivatisierten Metalloberflächen wurden nach Abdeckung mit einer Photomaske einer intensiven UV-Bestrahlung im Bereich der Absorptionsbanden einer Wellenlänge von 200 - 600 nm für 1 Sekunde bis 30 Minuten ausgesetzt. Danach wurde Glucoseoxidase gekoppelt. Die Aktivität der Glucoseoxidase wurde amperometrisch gemessen. Dabei zeigte sich, daß die belichteten Stellen der Oberfläche eine der Lichtmenge proportionale Desaktivierung der Kopplungsgruppen aufweisen. Die Menge der gekoppelten Glucoseoxidase war um einen der Lichtmenge proportionalen Wert geringer.

### Beispiel 5: Immobilisierung auf mit Verbindungen der Formel (I) derivatisierten Oberflächen

Die derivatisierten Trägermaterialien werden mit 5(w/w) des zu immobilisierenden Proteins in der 10 - 20fachen Menge eines geeigneten Puffers bei einem pH-Wert im Bereich von 4 - 9,2 Stunden bei Raumtemperatur inkubiert. Das Immobilisat wurde durch Waschen mit unterschiedlichen Puffern von adsorbierenden Protein befreit.

### A.

Ein Naringenaseimmobilisat ausgehend von nativer Naringenase mit 1 nKAT/mg wies eine Aktivität von 5,5 nKAT/g Immobilisat auf.

### B.

Ein Phosphataseimmobilisat ausgehend von nativer alkalischer Phosphatase mit 0,25 nKAT/mg wies eine Aktivität von 2 nKAT/g Immobilisat auf.

### C.

Ein β-Galactoxidaseimmobilisat ausgehend von nativer β-Galactoxidase mit 0,68 nKAT/mg wies eine Aktivität von 4,6 nKAT/g Immobilisat auf.

### D.

Technische Lipase aus Candida cyclindracea zeigte gleiche Synthetase und Hydrolase-Aktivität wie vergleichbare Immobilisate mit Glutardialdehyd jedoch geringes Klumpenverhalten in organischen Lösungsmitteln.

### E.

Die Aktivität auf Metalloberflächen immobilisierter Glucoseoxidase entsprach einer dichten monomolekularen Enzymschicht.

### Beispiel 6: Immobilisierung auf mit Verbindungen der Formel (IA) derivatisierten Oberflächen

Die derivatisierten Trägermaterialien werden mit 15(w/w) des zu immobilisierenden Proteins in der 10 - 20fachen Menge eines geeigneten Puffers bei einem pH-Wert in einem Bereich von 4 - 9,2 Stunden bei 4 - 25°C inkubiert. Das Immobilisat wurde durch Waschen mit unterschiedlichen Puffern von adsorbiertem Protein befreit.

### A.

Ein Glucuronidaseimmobilisat, ausgehend von nativer Glucuronidase mit 330 nKAT/g, wies eine Aktivität von 22 nKAT/g Immobilisat auf. Der Proteingehalt betrug 51 mg/g Träger.

### B.

Ein alkalisches Phosphataseimmobilisat, ausgehend von nativer Phosphatase mit 26 KAT/g, wies eine Aktivität von 420 nKAT/g Immobilisat auf. Der Proteingehalt betrug 20,6 mg/g Träger.

### C.

Ein β-Galactosidaseimmobilisat, ausgehend von nativer Galactosidase mit 7,6 KAT/g, wies eine Aktivität von 43 nKAT/g Immobilisat auf.

## Patentansprüche

1. Verfahren zum Immobilisieren von Proteinen, Peptiden, Liganden, Coenzymen oder Redoxmeditaoren an einem Träger, welcher Amino-, Mercapto- oder Hydroxygruppen aufweist, dadurch gekennzeichnet, daß der Träger mit einer Verbindung der allgemeinen Formel (I) oder (IA) 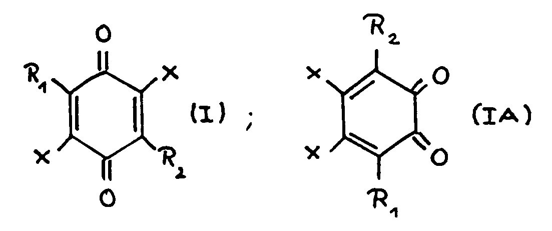 in welchen X Halogen bedeutet und R₁, R₂ gleich oder ungleich sind und X, R, COR, COOR, worin R C₁ - C₈ Alkyl bedeutet, COOH, CNS, N₃ oder CN bedeuten, umgesetzt und gewünschtenfalls getrocknet wird, worauf die zu immobilisierenden Peptide, Proteine oder Coenzyme mit dem zur C=O Gruppe ortho- oder paraständigen, freien Halogen der Verbindung der allgemeinen Formel (I) oder (IA) umgesetzt werden.

2. Verfahren nach Anspruch 1, dadurch gekennzeichnet, daß als Verbindung der allgemeinen Formel (I) 2,3,5,6-Tetrachlorcyclohexadien-1,4-dion eingesetzt wird.

3. Verfahren nach Anspruch 1, dadurch gekennzeichnet, daß als Verbindung der allgemeinen Formel (IA) 3,4,5,6-Tetrachlorcyclohexadien-1,2-dion oder 3,4,5,6 - Tetrabromcyclohexadien-1,2-dion eingesetzt wird.

4. Verfahren nach Anspruch 1 oder 2, dadurch gekennzeichnet, daß die Umsetzung des Trägers mit Verbindungen der allgemeinen Formel (I) oder (IA) in organischen oder organisch wäßrigen Mischphasen vorgenommen wird.

5. Verfahren nach Anspruch 4, dadurch gekennzeichnet, daß die Umsetzung des Trägers mit Verbindungen der allgemeinen Formel (I) oder (IA) in wasserfreien, organischen Lösungsmitteln, insbesondere Toluol, vorgenommen wird.

6. Verfahren nach einem der Ansprüche 1 bis 5, dadurch gekennzeichnet, daß die Umsetzung der Verbindung der allgemeinen Formel (I) oder (IA) mit dem Träger bei Temperaturen zwischen -10°C und Rückflußtemperatur, vorzugsweise 20-70°C, vorgenommen wird.

7. Verfahren nach einem der Ansprüche 1 bis 6, dadurch gekennzeichnet, daß als Proteine Redoxenzyme, wie Glucoseoxidase, Galactoseoxidase, Aminosäureoxidase, Xanthinoxidase, Cholesterinoxidase, Uricase und Ascorbatoxidase bzw. deren Apoenzyme immobilisiert werden.

8. Verfahren nach einem der Ansprüche 1 bis 7, dadurch gekennzeichnet, daß Antigene oder Antikörper zum Immobilisieren eingesetzt werden.

9. Verfahren nach einem der Ansprüche 1 bis 8, dadurch gekennzeichnet, daß Naringinasen, α oder β-Glucosidasen, Glycosidasen, Galaktosidasen, Lipasen, Pectinasen, Esterasen, Penicillininase, Ammoniaklyasen, Urease und/oder Phosphatasen eingesetzt werden.

10. Verfahren nach einem der Ansprüche 1 bis 9, dadurch gekennzeichnet, daß die Immobilisierung zur Herstellung eines Biosensors auf einem mit Elektroden versehenen Träger erfolgt.

11. Verfahren nach einem der Ansprüche 1 bis 10, dadurch gekennzeichnet, daß die Oberfläche des Umsetzungsproduktes mit der Verbindung der allgemeinen Formel (I) oder (IA) teilweise durch photochemische Reaktion oder durch Anwendung alkalischer Reagentien bzw. niedermolekularer Amine oder Mercaptanen desaktiviert wird, worauf die Umsetzung mit den Peptiden, Proteinen, Coenzyme oder Liganden mit den verbleibenden Teilen der Oberfläche vorgenommen wird.

12. Verfahren nach einem der Ansprüche 1 bis 11, dadurch gekennzeichnet, daß das Immobilisat neuerlich mit einer Verbindung der allgemeinen Formel (I) oder (IA) umgesetzt wird, worauf gegebenenfalls nach teilweiser Desaktivierung der Oberfläche neuerlich Peptide, Proteine oder Coenzyme immobilisiert werden.

13. Verfahren nach einem der Ansprüche 1 bis 12, dadurch gekennzeichnet, daß das Immobilisat für die Verwendung als Biosensor mit einer physiologisch verträglichen Deckschicht, insbesondere mit einer negativen Raumladung, versehen wird.

14. Verfahren nach einem der Ansprüche 1 bis 13, dadurch gekennzeichnet, daß bei Verwendung des Immobilisates als Biosensor die Meßwerte elektrochemisch bestimmt werden.

15. Verfahren nach Anspruch 1, dadurch gekennzeichnet, daß Proteine, Peptide, Coenzyme oder Liganden mit einer wenigstens äquimolaren Menge einer Verbindung der Formel (I) oder (IA) 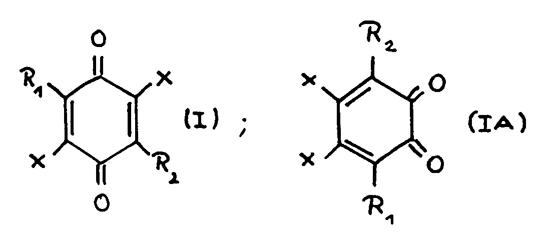 in welchen X Halogen bedeutet und R₁, R₂ gleich oder ungleich sind und X, R, COR, COOR, worin R C₁ - C₈ Alkyl bedeutet, COOH, CNS, N₃ oder CN bedeuten, umgesetzt werden und gegebenenfalls getrocknet werden, worauf das erhaltene Umsetzungsprodukt über ein freies zur C=O Gruppe ortho- oder paraständiges Halogen der Verbindung der Formel (I) oder (IA) mit dem Träger gekuppelt wird.

## Claims

1. A method for immobilizing proteins, peptides, ligands, coenzymes or redox mediators on a carrier containing amino, mercapto or hydroxy groups, characterized in that the carrier is reacted with a compound of the general formula (I) or (IA) 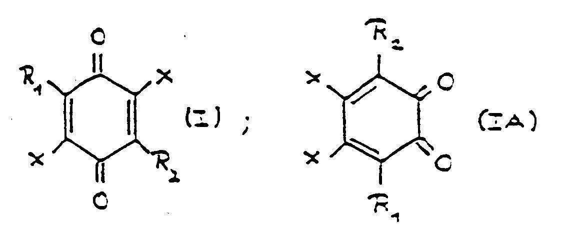 wherein X represents halogen and R₁, R₂ are the same or different, and represent X, R, COR, COOR, wherein R represents C₁ - C₈ alkyl, COOH, CNS, N₃ or CN, and, optionally, dried, after which the proteins, peptides or coenzymes to be immobilized are reacted with the free halogen of the compound of the general formula (I) or (IA), which is in an ortho or para position in relation to the C=O group.

2. A method as claimed in Claim 1, characterized in that 2,3,5,6-tetrachlorocyclohexadiene-1,4-dion is used as the compound of the general formula (I).

3. A method as claimed in Claim 1, characterized in that 3,4,5,6-tetrachlorocyclohexadiene-1,2-dion or 3,4,5,6-tetrabromocyclohexadiene-1,2-dion is used as the compound of the general formula (IA).

4. A method as claimed in either of claims 1 or 2, characterized in that reaction of the carrier with compounds of the general formula (I) or (IA) is conducted in organic or organically aqueous mixed phases.

5. A method as claimed in Claim 4, characterized in that the reaction of the carrier with compounds of the general formula (I) or (IA) is conducted in non-aqueous, organic solvents, especially toluene.

6. A method as claimed in any of the claims 1 to 5, characterized in that the reaction of the compound of the general formula (I) or (IA) with the carrier is conducted at temperatures between -10 °C and reflux temperature, preferably 20 - 70 °C.

7. A method as claimed in any of the claims 1 to 6, characterized in that redox enzymes such as glucose oxidase, galactose oxidase, amino-acid oxidase, xanthine oxidase, cholesterol oxidase, uricase or ascorbate oxidase or their apoenzymes are immobilized as proteins.

8. A method as claimed in any of claims 1 to 7, characterized in that antigens or antibodies are used in immobilization.

9. A method as claimed in any of the claims 1 to 8, characterized in that naringinases, α or β-glucosidases, glycosidases, galactosidases, lipases, pectinases, esterases, penicillininase, ammonialyases, urease and/or phosphatases are used.

10. A method as claimed in any of claims 1 to 9, characterized in that immobilization is conducted for the production of a biosensor on a carrier equipped with electrodes.

11. A method as claimed in any of the claims 1 to 10, characterized in that the surface of the reaction product is partially deactivated with the compound of the general formula (I) or (IA) by means of a photochemical reaction or by using alkaline reagants or low-molecular amines or thiols, after which reaction with the peptides, proteins, coenzymes or ligands with the remaining sections of the surface is conducted.

12. A method as claimed in any of the claims 1 to 11, characterized in that the immobilizate is reacted again with a compound of the general formula (I) or (IA), after which peptides, proteins or coenzymes are again immobilized optionally after partial deactivation of the surface.

13. A method as claimed in any of the claims 1 to 12, characterized in that the immobilizate is provided with a physiologically acceptable coating and especially with a negative space charge for use as a biosensor.

14. A method as claimed in any of the claims 1 to 13, characterized in that the measured values are determined electrochemically during use of the immobilizate as a biosensor.

15. A method as claimed in Claim 1, characterized in that proteins, peptides, coenzymes or ligands are reacted with at least an equimolar amount of a compound of the formula (I) or (IA) 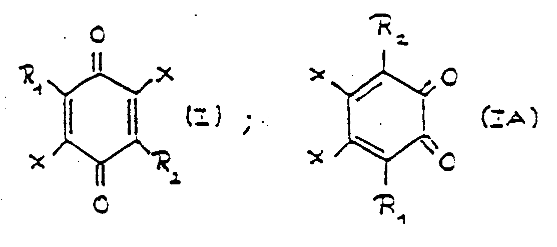 wherein X represents halogen and R₁, R₂ are the same or different, and represent X, R, COR, COOR, wherein R represents C₁ - C₈ alkyl, COOH, CNS, N₃ or CN, and, optionally, dried, after which the reaction product obtained is coupled to the carrier via a free halogen of the compound of the formula (I) or (IA), which is in an ortho or para position in relation to the C=O group.

## Revendications

1. Procédé d'immobilisation de protéines, peptides, ligands, coenzymes ou médiateurs redox sur un support comportant des groupes amine, mercapto ou hydroxy, caractérisé en ce que l'on fait réagir le support avec une combinaison de la formule générale (I) ou (IA), 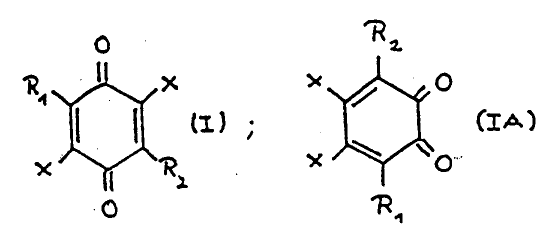 dans lesquelles X représente un halogène et R₁, R₂ sont identiques ou différents, et signifient X, R, COR, COOR, avec R représentant C₁ - C₈ alkyle, COOH, CNS, N₃ ou CN, à le sécher, le cas échéant, et par conséquent à faire réagir les peptides, protéines ou coenzymes à immobiliser avec l'halogène libre en position ortho ou para par rapport au groupe C=O de la combinaison de la formule générale (I) ou (IA).

2. Procédé selon revendication 1 caractérisé en ce que l'on utilise, code combinaison de la formule générale (I), le 2,3,5,6-tétrachlorocyclohexadiène-1,4-dion.

3. Procédé selon revendication 1 caractérisé en ce que l'on utilise, comme combinaison de la formule générale (I), le 3,4,5,6-tétrachlorocyclohexadiène-1,2-dion ou 3,4,5,6-tétrabromocyclohexadiène-1,2-dion.

4. Procédé selon revendication 1 ou 2, caractérisé en ce que la réaction du support est réalisée avec des combinaisons de la formule générale (I) ou (IA) dans des phases mixtes organiques ou organo-aqueuses.

5. Procédé selon revendication 4, caractérisé en ce que la réaction du support est réalisée avec des combinaisons de la formule générale (I) ou (IA) dans des solvants anhydres organiques, en particulier du toluène.

6. Procédé selon l'une des revendications 1 à 5, caractérisé en ce que la réaction de la combinaison de la formule générale (I) ou (IA) avec le support est réalisée à des températures situées entre -10°C et la température de reflux, de préférence 20-70°C.

7. Procédé selon l'une des revendications 1 à 6, caractérisé en ce que les protéines immobilisées sont des enzymes redox tels que la glucose-oxydase, la galactose-oxydase, l'aminoacide-oxydase, la xanthine-oxydase, la cholestérol-oxydase, l'uricase et l'ascorbate-oxydase ou leurs apoenzymes.

8. Procédé selon l'une des revendications 1 à 7, caractérisé en ce que, pour l'immobilisation, des antigènes ou des anticorps sont mis en oeuvre.

9. Procédé selon l'une des revendications 1 à 8, caractérisé en ce que les enzymes utilisés sont des naringinases, des alpha ou beta-glucosidases, des glycosidases, ds galactosidases, des lipases, des pectinases, des esterases, des pénicillinases, des ammoniac-lyases, des uréases et/ou des phosphatases.

10. Procédé selon l'une des revendications 1 à 9, caractérisé en ce que l'immobilisation en vue de la production d'un biodétecteur est réalisée sur un support pourvu d'électrodes.

11. Procédé selon l'une des revendications 1 à 10, caractérisé en ce que la surface du produit de réaction avec la combinaison de la formule générale (I) ou (IA) est désactivée en partie par réaction photochimique ou par utilisation de réactifs alcalins ou d'amines ou de mercaptans de faible poids moléculaire et que par la suite la réaction avec les peptides, protéines, coenzymes ou ligands est réalisée avec les parties rémanentes de la surface.

12. Procédé selon l'une des revendications 1 à 11, caractérisé en ce que l'immobilisat subit une nouvelle réaction avec une combinaison de la formule générale (I) ou (IA), avec obtention, le cas échéant, après désactivation partielle de la surface, d'une nouvelle immobilisation de peptides, protéines ou coenzymes.

13. Procédé selon l'une des revendications 1 à 12, caractérisé en ce que l'immobilisat destiné à une utilisation comme biodétecteur est doté d'une couche de surface de compatibilité physiologique, en particulier de charge spatiale négative.

14. Procédé selon l'une des revendications 1 à 13, caractérisé par le fait que l'immobilisat est destiné à une utilisation comme biodétecteur, les valeurs de mesures sont déterminées par voie électrochimique.

15. Procédé selon revendication 1, caractérisé en ce que l'on fait réagir des protéines, des peptides, des coenzymes ou des ligands avec au moins une quantité équimoléculaire d'une combinaison de la formule I ou (IA) 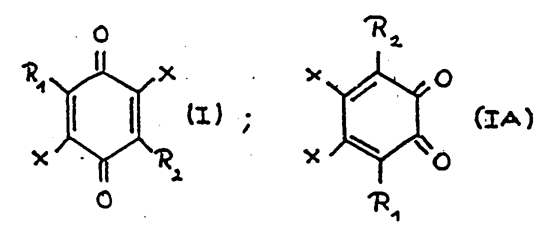 dans lesquelles X représente un halogène, où R₁, R₂ sont identiques ou différents, et signifient X, R, COR, COOR, avec R représentant C₁ - C₈ alkyle, COOH, CNS, N₃ ou CN, à le sécher, le cas échéant, et par la suite, à coupler le produit de réaction avec le support par le biais d'un halogène libre en position ortho ou para par rapport au groupe C=O de la combinaison de la formule (I) ou (IA).
